(19) Europäisches Patentamt
European Patent Office
Office européen des brevets

(11) EP 2 842 965 A1

(12) **EUROPEAN PATENT APPLICATION**

(43) Date of publication:
**04.03.2015 Bulletin 2015/10**

(21) Application number: **13182749.5**

(22) Date of filing: **03.09.2013**

(51) Int Cl.:
***C07K 14/575*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Gubra ApS**
**2970 Horsholm (DK)**

(72) Inventors:
- **Pedersen, Søren Ljungberg**
  **4140 Borup (DK)**
- **Dalbøge, Louise Schjellerup**
  **2100 Copenhagen Ø (DK)**
- **van Witteloostuijn, Søren Blok**
  **2400 Copenhagen NV (DK)**
- **Hansen, Henrik Björk**
  **2750 Ballerup (DK)**
- **Jelsing, Jacob**
  **4000 Roskilde (DK)**
- **Vrang, Niels**
  **2930 Klampenborg (DK)**

(74) Representative: **Bender, Mikkel**
**COPA Copenhagen Patents**
**Vesterbrogade 10, 2nd floor**
**1620 Copenhagen V (DK)**

(54) **Neuromedin U analogs comprising serum albumin binding amino acid residue**

(57) The present invention relates to neuromedin U receptor agonist peptides comprising at least one serum albumin binding amino acid residue, said serum albumin binding amino acid residue being distanced by at least one but less than 16 amino acids from the part of the peptide comprising the active site responsible for NMU receptor binding, as well as to pharmaceutical and veterinary compositions comprising the peptides.

EP 2 842 965 A1

## Description

Reference to a Sequence Listing

This application contains a Sequence Listing in computer readable form, which is incorporated herein by reference.

Technical Field

The present invention relates to peptide analogs of neuromedin U (NMU) comprising one or more serum albumin binding modifications, as well as to the use thereof for treatment of metabolic disorders. The present invention also relates to pharmaceutical or veterinary compositions comprising the peptide analogs of neuromedin U.

Background

Neuromedin U (NMU) is a conserved neuropeptide present in many species, existing as multiple isoforms. The sequence of Human neuromedin U is H-FRVDEEFQSPFASQSRGYFLFRPRN-$NH_2$, of which the C-terminal octapeptide consisting of the residues YFLFRPRN has been shown to constitute the active site, i.e. the part of the NMU peptide being responsible for the activity of NMU in binding the NMU receptors in the human body. This part of the NMU sequence is highly conserved within animal species. Especially the seven C-terminal residues (i.e. the residues FLFRPRN) have been shown to be identical across all species that have been examined and these are considered to be critical for bioactivity (Brighton et al, Pharmacol. Rev. 56: 231-248 (2004)). Substitutions in the first amino acid residue (phenylalanine) with tyrosine are known from rat NMU, and substitution for glutamine is known from dog NMU. Further, several NMU analogs having activity as NMU receptor agonists are known, including analogs having alterations in the C-terminal octapeptide. Funes et al. have reported an Ala-scan of porcine NMU8 (C-terminal octapeptide), which indicated that all amino acid residues of NMU8 are important for in vitro activity, with Arginine at position 5 (R5) and 7 (R7) being the most important contributors (Funes et al., Peptides 23: 1607-1615 (2002)). Hashimoto et al. has also shown Phenylalanine at positions 2 (F2) and 4 (F4), Arginine at position 5 (R5) and 7 (R7), Proline at position 6 (P6), and Asparagine at position 8 (N8) to be important for porcine NMU8 and canine NMU8 (Chem. Pharm Bull. 39; 2319-2322 (1991), Chem. Pharm. Bull. 43; 1148-1153 (1995), Chem. Pharm. Bull. 43; 1154-1157 (1995), Chem. Pharm. Bull. 44; 1180-1884 (1996), Chem. Pharm. Bull. 48; 1166-1170 (2000)). Nevertheless WO 2011005611 A1 and WO 2007109135 A2 describe that Asparagine at position 8 can be replaced with D-Alanine, Glycine or D-Norleucine (D-Nle) and Arginine at position 7 can be replaced with homoArginine. Moreover, WO 2007109135 A2 describes that Tyrosine at position 1 and Phenylalanine at position 2 can be replaced by Phenylalanine at position 1 and Alanine at position 2. US 20120094898 A1 describes that Phenylalanine at position 2 can be replaced by 2-Nal (β-(2-Naphthyl)-alanine).

The activation of NMU receptors leads to intracellular signal transduction via calcium mobilization, phosphoinositide (or PI) signalling, and the inhibition of cAMP production. Two NMU receptors have been identified: NMUR1, expressed primarily in the periphery, and NMUR2, expressed predominantly in the brain.

The role of Neuromedin U (NMU) in the regulation of feeding and energy homeostasis is well documented. It is known that NMU exerts potent anorectic activity and can improve glucose homeostasis, with both actions believed to be mediated by NMUR1.

Thus, NMU and analogs thereof are known to be effective in e.g. regulation and treatment of metabolic disorders, such as diabetes and obesity.

However, the *in vivo* activity of NMU and NMU analogs is often compromised due to degradation of the peptide *in vivo.*

Thus, there is a need in the art for NMU analogs having increased and/or prolonged in *vivo* activity.

Several analogs of NMU have been proposed and produced, in which the *in vivo* activity of the peptide is increased, by the placement of functional groups on the peptides. These functional groups presumably provide protection against degradation *in vivo.* Especially NMU analogs comprising poly(ethylene) glycol (PEG) modifications are well known and well-studied. Addition of poly(ethylene) glycol (PEG) is often termed "PEGylation". The covalent attachment of PEG to a drug or therapeutic protein/peptide can mask the agent from the host’s immune system (reduced immunogenicity and antigenicity), and increase the hydrodynamic volume (size in solution) of the agent which prolongs its circulation time by reducing its renal clearance. PEGylation can also provide water solubility to hydrophobic drugs, peptides, and proteins.

Ingallinella et al, Bioorganic & Medicinal Chemistry 20 (2012) 4751-4759, describe the development of a metabolically stable analog of NMU, based on derivatization of the native peptide with high molecular weight poly(ethylene) glycol (PEG). The authors state that the optimal placement of the PEG protection group is at the N-terminus of the NMU analogs, and conclude that derivatization of NMU at the N-terminus with a 40 kDa PEG represents the most appropriate

solution to achieve metabolic stability and prolonged duration of action.

Another strategy for modifying NMU to provide increased and/or prolonged *in vivo* activity is by addition of a functional group providing binding to serum albumin. Addition of such functional group in the form of a lipid providing binding to serum albumin, is often termed "lipidation".

WO 2011005611 A1 describes several modified NMU analogs, and discloses the preference of using NMU analogs comprising N-terminal serum albumin binding amino acid analogs (as seen in the experimental part, and as claimed in claim 1 of WO 2011005611 A1). NMU34 is a palmitoylated analog in which the palmitoyl group is linked at the N- terminus of the C-terminal nonamer of the NMU peptide sequence. NMU44 is a similar cholesteroylated peptide. The analogs NMU34 and NMU44 disclosed in WO 2011005611 A1 are thus NMU analogs comprising a serum albumin binding amino acid positioned N-terminal at a distance of one amino acid from the active site (the sequence of SEQ ID NO: 1). Further disclosed serum albumin binding amino acid analogs are NMU analogs NMU38, NMU39 and NMU40. NMU38 is a palmitoylated analog in which the palmitoyl group is linked at the N-terminus of the NMU peptide sequence. NMU39 and NMU40 are palmitoylated analogs in which a Ttds (1-amino-4,7,10-trioxa-13-tridecanamine succinimic acid) or a gamma-glutamic acid residue have been introduced as flexible spacers between the N-terminus of the NMU sequence and the palmitoyl group. Thus, NMU38, NMU39 and NMU40 are NMU analogs which comprise a serum albumin binding amino acid positioned N-terminal at a distance of 16 amino acids from the active site (SEQ ID NO: 1) of NMU. Further, it is disclosed that analogs (e.g. NMU38) wherein the serum albumin binding amino acid is distanced by 16 amino acids from the active site of NMU provide the most promising results.

However, there is a constant need in the art for providing NMU analogs having increased activity *in vivo*.

Thus, it was an object of the present invention to provide analogs of NMU with increased activity *in vivo*.

There is further a constant need in the art for providing NMU analogs having increased activity in *vivo* when dosed at low concentrations.

Thus, it was a further object of the invention to provide analogs of NMU with increased activity *in vivo* when dosed at low concentrations.

There is further a constant need in the art for providing NMU analogs having prolonged activity *in vivo*.

Thus, it was a further object of the invention to provide NMU analogs with prolonged activity *in vivo.*

There is further a constant need in the art for providing NMU analogs having prolonged activity in vivo when dosed at low concentrations.

Thus, it was a further object of the invention to provide analogs of NMU with prolonged activity *in vivo* when dosed at low concentrations.

Summary of the invention

Until the present invention, it is believed in the art that NMU analogs are most effective when placing protective groups in the N-terminal part of the peptide, well distanced from the active site of the peptide. This general belief was further confirmed during the *in vitro* experiments leading to the present invention. Further, it was demonstrated that placement of a serum albumin binding group near the active site of NMU (the active site having the sequence YFLFRPRN (SEQ ID NO: 1)) leads to a decreased activity of the peptide *in vitro.*

The present invention, however, is based on the surprising finding that the *in vivo* activity of NMU analogs comprising a serum albumin binding group increases, when the serum albumin binding group is placed near the active site (i.e. placed near the C-terminal part of the NMU analog), albeit at a distance of at least one amino acid residue. Further, it was demonstrated that N-terminal placement of the serum albumin binding group was not of essential importance.

These surprising effects are especially pronounced, when the analogs are provided at low dosages.

Accordingly, it was surprisingly found that placement of an amino acid comprising a serum albumin binding group at a distance of at least 1 but less than 16 amino acid residues from the active site in NMU leads to an increased activity of the peptide, especially when dosed at low concentrations.

Thus, in one aspect, the present invention relates to a Neuromedin U receptor agonist peptide comprising a first C-terminal part and a second N-terminal part, said first part comprising an amino acid sequence corresponding to the sequence of SEQ ID NO: 1;

YFLFRPRN (SEQ ID NO: 1) or a functional analog thereof,

said second part comprising at least two amino acid residues wherein at least one amino acid residue is a serum albumin binding amino acid residue, said serum albumin binding amino acid residue being distanced by at least 1 but less than 16 amino acid residues from the first part of the peptide, as well as to pharmaceutical and veterinary compositions comprising such peptide.

In another aspect the present invention relates to the use of a Neuromedin U receptor agonist peptide comprising

a first C-terminal part and a second N-terminal part, said first part comprising an amino acid sequence corresponding to the sequence of SEQ ID NO: 1;

YFLFRPRN (SEQ ID NO: 1) or a functional analog thereof,

said second part comprising at least two amino acid residues wherein at least one amino acid residue is a serum albumin binding amino acid residue, said serum albumin binding amino acid residue being distanced by at least 1 but less than 16 amino acid residues from the first part of the peptide for medical or veterinary treatment of a human or animal subject, especially for the treatment of metabolic disorders such as diabetes and obesity.

Definitions

"Sequence identity":

The relatedness between two amino acid sequences or between two nucleotide sequences is described by the parameter "sequence identity". For purposes of the present invention, the sequence identity between two amino acid sequences may be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277). The parameters used are gap open penalty of 10, gap extension penalty of 0.5, and the EBLOSUM62 (EMBOSS version of BLOSUM62) substitution matrix. The output of Needle labelled "longest identity" (obtained using the - no brief option) is used as the percent identity and is calculated as follows:

$$(\text{Identical Residues x } 100)/(\text{Length of Alignment} - \text{Total Number of Gaps in Alignment}).$$

"Sequence identity of at least 70%":

It should be generally understood that according to the present invention, whenever the phrase "sequence identity of at least 70%" is used, it should mean a sequence identity of 70% or higher compared to the respective peptide as described above, the higher sequence identity being preferred. Accordingly, it should be understood that "sequence identity of at least 70%" includes a preferred embodiment having a sequence identity of at least 71%, such as a sequence identity of at least 72%, such as a sequence identity of at least 73%, such as a sequence identity of at least 74%, such as a sequence identity of at least 75%, such as a sequence identity of at least 76%, such as a sequence identity of at least 77%, such as a sequence identity of at least 78%, such as a sequence identity of at least 79%, such as a sequence identity of at least 80%, such as a sequence identity of at least 81%, such as a sequence identity of at least 82%, such as a sequence identity of at least 83%, such as a sequence identity of at least 84%, such as a sequence identity of at least 85%, such as a sequence identity of at least 86%, such as a sequence identity of at least 87%, such as a sequence identity of at least 88%, such as a sequence identity of at least 89%, such as a sequence identity of at least 90%, such as a sequence identity of at least 91%, such as a sequence identity of at least 92%, such as a sequence identity of at least 93%, such as a sequence identity of at least 94%, such as a sequence identity of at least 95%, such as a sequence identity of at least 96%, such as a sequence identity of at least 97%, such as a sequence identity of at least 98%, such as a sequence identity of at least 99%, such as a sequence identity of 100% compared to the respective peptide as described above.

"Amino acid":

According to the present invention, amino acid means any amino acid such as a natural or non-natural amino acid. Preferred amino acids according to the present invention are natural amino acids, i.e. Alanine (A), Arginine (R), Asparagine (N), Aspartic acid (D), Cysteine (C), Glutamic acid (E), Glutamine (Q), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), Valine (V). Further, natural amino acids comprise Selenocysteine (U) and Pyrrolysine (O). Naturally occurring amino acids, which are not encoded by the genetic code, include i.e. $\gamma$-carboxyglutamate, ornithine, hydroxyproline (Hyp), phosphoserine, D-alanine and D-glutamine. Non-natural amino acids comprise artificial amino acids manufactured by chemical synthesis, i.e. D-isomers of the amino acids encoded by the genetic code such as D-alanine and D-leucine, $\alpha$-aminoisobutyric acid (Aib), $\alpha$-aminobutyric acid (Abu), tert-butylglycine (Tle), $\beta$-amino acids such as $\beta$-alanine, and 3-aminomethyl benzoic acid, anthranilic acid, homoarginine (hArg), 3-mercaptophenylalanine, 2- or 3-hydroxyphenylalanine (*o*- or *m*-tyrosine), hydroxymethylglycine and aminoethylglycine. When linked together in chains (peptides), each amino acid loses a water molecule and becomes an amino acid residue. Amino acids and amino acid residues are, in the context of the present invention, used interchangeably.

"Serum albumin binding amino acid":

According to the present invention, serum albumin binding amino acid means an amino acid such as a natural or non-natural amino acid which has been modified by attaching a serum albumin binding side chain to the amino acid backbone. The attachment of a serum albumin binding side chain to the amino acid backbone may also be termed "acylation" .

"Serum albumin binding side chain":

According to the present invention, serum albumin binding side chain means a side chain attached to an amino acid (residue), the side chain consisting of or comprising a functional group capable of binding to serum albumin. A functional group is capable of binding to plasma proteins according to the present invention if the concentration of free peptide (the peptide comprising the functional group) is reduced in a plasma protein binding assay using rapid equilibrium dialysis and LC-MS/MS. Another example of a method for determination of albumin binding is by measuring the binding of the peptide using enzyme-linked immunosorbent assay (ELISA) using a microtiter plate with immobilised albumin as described in US 20110275559 A1. In a preferred embodiment of the present invention, the serum albumin binding side chain comprises a lipid molecule (termed lipidated amino acid), cholesterol molecule or sialic acid molecules.

"Functional analog of amino acid sequence corresponding to the sequence of SEQ ID NO: 1":

According to the present invention, a "functional analog of amino acid sequence corresponding to the sequence of SEQ ID NO: 1" means a peptide or peptide sequence capable of binding and activating a NMU receptor present in the human or animal body, i.e. capable of binding and activating the NMUR1 receptor as determined using the "functional assay" described in the examples of the present specification, and giving a result of an $EC_{50}$ value of less than 1000 nM, and/or capable of binding and activation the NMUR2 receptor as determined using the "functional assay" described in the examples of present specification, and giving a result of an $EC_{50}$ value of less than 1000 nM.

An alternative definition of a "functional analog of amino acid sequence corresponding to the sequence of SEQ ID NO: 1" is an analog having least 60% sequence identity with the peptide of SEQ ID NO: 1. Even more specifically, the "functional analog of amino acid sequence corresponding to the sequence of SEQ ID NO: 1" is an analog having at least 65% sequence identity with the peptide of SEQ ID NO: 1. Even more specifically, the "functional analog of amino acid sequence corresponding to the sequence of SEQ ID NO: 1" is an analog having at least 70% sequence identity with the peptide of SEQ ID NO: 1. Even more specifically, the "functional analog of amino acid sequence corresponding to the sequence of SEQ ID NO: 1" is an analog having at least 75% sequence identity with the peptide of SEQ ID NO: 1. Even more specifically, the "functional analog of amino acid sequence corresponding to the sequence of SEQ ID NO: 1" is an analog having at least 80% sequence identity with the peptide of SEQ ID NO: 1. Even more specifically, the "functional analog of amino acid sequence corresponding to the sequence of SEQ ID NO: 1" is an analog having at least 85% sequence identity with the peptide of SEQ ID NO: 1.

"Being distanced by at least one but less than 16 amino acid residues":

According to the present invention, the phrase "being distanced by at least one but less than 16 amino acid residues" is to be understood as the respective groups "being distanced by at least one but less than 16 amino acid residues", i.e. connected in one and the same peptide, the connection consisting of at least 1 amino acid residue and less than 16 amino acid residues.

<u>Detailed description of the Invention</u>

During the experiments leading to the present invention it was surprisingly observed that the *in vivo* activity of NMU analogs comprising a serum albumin binding side chain increased as the amino acid residue comprising the modification was placed closer to the active site of the NMU analog.

Thus, in a first aspect the present invention relates to a Neuromedin U receptor agonist peptide comprising a first C-terminal part and a second N-terminal part, said first part comprising an amino acid sequence corresponding to

the sequence of SEQ ID NO: 1;

YFLFRPRN (SEQ ID NO: 1) or a functional analog thereof,

said second part comprising at least two amino acid residues wherein at least one amino acid residue is a serum albumin binding amino acid residue, said serum albumin binding amino acid being distanced by at least 1 but less than 16 amino acids from the first part of the peptide.

In a preferred embodiment, the first C-terminal part of the peptide comprising an amino acid sequence corresponding to the sequence of SEQ ID NO: 1 consists of the seven amino acids FLFRPRN as well as up to one amino acid residue selected among tyrosine, phenylalanine and glutamine, preferably tyrosine, N-terminal of the seven amino acids, and may comprise additional amino acid residues C-terminal of the seven amino acids.

In one embodiment of the present invention, the functional analog of SEQ ID NO: 1 comprises a substitution wherein the one amino acid residue N-terminal of the seven amino acids is a phenylalanine (F).

Preferably, the first part and the second part are directly connected and together form a peptide consisting of a first and a second part as defined in the present specification.

The second part comprises at least two amino acid residues wherein at least one amino acid residue is a serum albumin binding amino acid residue, said serum albumin binding amino acid residue being distanced by at least 1 amino acid residue from the first part of the peptide.

The functional analog of the first part amino acid sequence is preferably an amino acid sequence which corresponds to a peptide, which in itself, i.e. independently of further amino acids, is capable of binding one or more human NMU receptors with and EC50 of less than 1000 nM as determined using the "functional assay" described in the examples of the present specification.

In a preferred embodiment, the NMU analog peptide according to the invention comprises a first part having at least 60% sequence identity with the peptide of SEQ ID NO: 1. Preferably, the NMU analog peptide according to the invention comprise a first part having at least 65% sequence identity with the peptide of SEQ ID NO: 1. Even more preferably, the NMU analog peptide according to the invention comprise a first part having at least 70% sequence identity with the peptide of SEQ ID NO: 1. Even more preferably, the NMU analog peptide according to the invention comprise a first part having at least 75% sequence identity with the peptide of SEQ ID NO: 1. Even more preferably, the NMU analog peptide according to the invention comprise a first part having at least 80% sequence identity with the peptide of SEQ ID NO: 1. Even more preferably, the NMU analog peptide according to the invention comprise a first part having at least 80% sequence identity with the peptide of SEQ ID NO: 1, such as at least 81%, 82%, 83%, 84%, 85%, 86%, 87%, 88%, 89% sequence identity with the peptide of SEQ ID NO: 1. Even more preferably, the NMU analog peptide according to the invention comprises a first part having at least 90% sequence identity with the peptide of SEQ ID NO: 1, such as at least 91%, 92%, 93%, 94%, 95%, 96%, 97%, 98%, 99% sequence identity with the peptide of SEQ ID NO: 1.

In a preferred embodiment, the NMU analog peptide according to the invention comprises a first part as described above and a second part comprising a serum albumin binding amino acid residue, where the first part and the second part together correspond to a peptide sequence of SEQ ID NO: 2

$X_1X_2X_3X_4X_5X_6X_7X_8X_9X_{10}X_{11}X_{12}X_{13}X_{14}X_{15}X_{16}X_{17}$YFLFRPRN (SEQ ID NO: 2)

wherein each X may be absent or represent a non-serum albumin binding amino acid residue, with the proviso that the second part, i.e. the amino acid residues $X_1X_2X_3X_4X_5X_6X_7X_8X_9X_{10}X_{11}X_{12}X_{13}X_{14}X_{15}X_{16}X_{17}$ together, comprises at least two amino acid residues, characterised in that the second part of the peptide comprises at least one alteration at any one of the positions corresponding to positions $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$ or $X_{16}$ of the peptide of SEQ ID NO: 2, wherein the alteration is a substitution of the respective amino acid residue with a serum albumin binding amino acid residue or an insertion of a serum albumin binding amino acid residue. In a preferred embodiment, the NMU analog peptide according to the invention comprises a first part as described above and a second part comprising a serum albumin binding amino acid residue, where the first part and the second part together correspond to a polypeptide having at least 70% sequence identity with the peptide of SEQ ID NO: 3

$X_1X_2X_3X_4X_5X_6X_7X_8X_9X_{10}X_{11}X_{12}X_{13}X_{14}S_{15}R_{16}G_{17}$YFLFRPRN (SEQ ID NO: 3)

wherein each X may be absent or represent a non-serum albumin binding amino acid, characterised in that the peptide comprises an alteration at any one of the positions corresponding to positions $X_2$, $X_3$, $X_4$, $X_5$, $X_6$, $X_7$, $X_8$, $X_9$, $X_{10}$, $X_{11}$, $X_{12}$, $X_{13}$, $X_{14}$, $S_{15}$, or $R_{16}$ of the peptide of SEQ ID NO: 2, wherein the alteration is a substitution of the amino acid with a serum albumin binding amino acid or an insertion of a serum albumin binding amino acid.

In a preferred embodiment, the NMU analog peptide according to the invention comprises a first part as described above and a second part comprising a serum albumin binding amino acid residue, where the first part and the second part together correspond to a polypeptide having at least 70% sequence identity with the peptide of SEQ ID NO: 3

$X_1X_2X_3X_4X_5X_6X_7X_8X_9X_{10}X_{11}X_{12}X_{13}X_{14}S_{15}R_{16}G_{17}$YFLFRPRN (SEQ ID NO: 3)
is $X_2$ is R, K, Ornithine, Citrulline, N-methyl Arginine, N-methyl Lysine, N-methyl ornithine, Methyl lysine, Dimethyl lysine, Trimethyl lysine, 2-Amino-3-guanidino-propionic acid, 2-Amino-4-guanidino-butyric acid or Monomethyl arginine, Dimethyl arginine, (2-Guanidino-ethylamino)-acetic acid, (3-Guanidino-propylamino)-acetic acid, (4-Guanidino-butylamino)-acetic acid, 2-Amino-3-(4-guanidino-phenyl)propionic acid, 2,3-Diaminopropionic acid, 2,4-Diaminobutyric acid or absent; $X_3$ is V, L, I, Aminoisobutyric acid, Homoleucine, Norleucine, 1-Aminobutyric acid, 2,3-Diaminopropionic acid, 2,4-Diaminobutyric acid, Ornithine, K or absent; $X_4$ is D, E, Homoglutamic acid, 2,3-Diaminopropionic acid, 2,4-Diaminobutyric acid, Ornithine, K or absent; $X_5$ is E, D, Homoglutamic acid, 2,3-Diaminopropionic acid, 2,4-Diaminobutyric acid, Ornithine, K or absent; $X_6$ is E, D, Homoglutamic acid, 2,3-Diaminopropionic acid, 2,4-Diaminobutyric acid, Ornithine, K or absent; $X_7$ is F, Y, Homophenylalanine, Homotyrosine, Phenylglycine, N-methyl Phenylalanine, N-methyl Tyrosine, 2,3-Diaminopropionic acid, 2,4-Diaminobutyric acid, Ornithine, K or absent; $X_8$ is Q, N, N-methyl Glutamine, N-methyl Asparagine, 2,3-Diaminopropionic acid, 2,4-Diaminobutyric acid, Ornithine, K or absent; $X_9$ is S, T N-methyl Serine, N-methyl Threonine, Homoserine, Homothreonine, O-methyl Threonine, O-methyl Serine, 2,3-Diaminopropionic acid, 2,4-Diaminobutyric acid, Ornithine, K or absent; $X_{10}$ is P, Hydroxyproline, 2,3-Diaminopropionic acid, 2,4-Diaminobutyric acid, Ornithine, K or absent; $X_{11}$, is F, Y, Homophenylalanine, Homotyrosine, Phenylglycine, N-methyl Phenylalanine, N-methyl Tyrosine, 2,3-Diaminopropionic acid, 2,4-Diaminobutyric acid, Ornithine, K or absent; $X_{12}$ is A, Aib, N-methyl Alanine 2,3-Diaminopropionic acid, 2,4-Diaminobutyric acid, Ornithine, K or absent; $X_{13}$ is S, T N-methyl Serine, N-methyl Threonine, Homoserine, Homothreonine, O-methyl Threonine, O-methyl Serine, 2,3-Diaminopropionic acid, 2,4-Diaminobutyric acid, Ornithine, K or absent; and $X_{14}$ is Q, N, N-methyl Glutamine, N-methyl Asparagine, 2,3-Diaminopropionic acid, 2,4-Diaminobutyric acid, Ornithine, K or absent;
characterised in that the peptide comprises an alteration at any one of the positions corresponding to positions $X_1$, $X_2$, $X_3$, $X_4$, $X_5$, $X_6$, $X_7$, $X_8$, $X_9$, $X_{10}$ $X_{11}$, $X_{12}$, $X_{13}$, $X_{14}$, $S_{15}$, or $R_{16}$ of the peptide of SEQ ID NO: 2, wherein the alteration is a substitution of the amino acid residue with a serum albumin binding amino acid residue or an insertion of a serum albumin binding amino acid residue.

In a preferred embodiment, the NMU analog peptide according to the invention comprises a first part as described above and a second part comprising a serum albumin binding amino acid residue, where the first part and the second part together correspond to a polypeptide having at least 70% sequence identity with the peptide of SEQ ID NO: 3

$X_1X_2X_3X_4X_5X_6X_7X_8X_9X_{10}X_{11}X_{12}X_{13}X_{14}S_{15}R_{16}G_{17}$YFLFRPRN (SEQ ID NO: 3)
wherein $X_2$ is R or absent, $X_3$ is V or absent, $X_4$ is D or absent, $X_5$ is E or absent, $X_6$ is E or absent, $X_7$ is F or absent, $X_8$ is Q or absent, $X_9$ is S or absent, $X_{10}$ is P or absent, $X_{11}$, is F or absent, $X_{12}$ is A or absent, $X_{13}$ is S or absent and $X_{14}$ is Q or absent;
characterised in that the peptide comprises an alteration at any one of the positions corresponding to positions $X_2$, $X_3$, $X_4$, $X_5$, $X_6$, $X_7$, $X_8$, $X_9$, $X_{10}$, $X_{11}$, $X_{12}$, $X_{13}$, $X_{14}$, $S_{15}$ or $R_{16}$ of the peptide of SEQ ID NO: 2, wherein the alteration is a substitution of the amino acid residue with a serum albumin binding amino acid residue or an insertion of a serum albumin binding amino acid residue.

In a preferred embodiment, the NMU analog peptide according to the invention comprises a polypeptide having at least 80% sequence identity with the peptide of SEQ ID NO: 3

$X_1X_2X_3X_4X_5X_6X_7X_8X_9X_{10}X_{11}X_{12}X_{13}X_{14}S_{15}R_{16}G_{17}$YFLFRPRN (SEQ ID NO: 3)
wherein each X may be absent or represent a non-serum albumin binding amino acid, characterised in that the agonist peptide comprises an alteration at one of the positions corresponding to positions S15, R16 and G17 of the peptide of SEQ ID NO: 3, wherein the alteration is a substitution of an amino acid residue with an amino acid residue having a serum albumin binding side chain or an insertion of an amino acid having a serum albumin binding side chain.

A highly preferred NMU analog peptide according to the invention comprises a polypeptide having the sequence

$X_1X_2X_3X_4X_5X_6X_7X_8X_9X_{10}X_{11}X_{12}X_{13}X_{14}K_{15}$RGYFLFRPRN (SEQ ID NO: 4)

wherein each X may be absent or represent a non-serum albumin binding amino acid, characterised in that the lysine$_{15}$ ($K_{15}$) amino acid residue comprises a serum albumin binding side chain.

A highly preferred NMU analog peptide according to the invention comprises a polypeptide having the sequence

FRVDEEFQSPFASQKRGYFLFRPRN (SEQ ID NO: 5)

characterised in that the lysine$_{15}$ ($K_{15}$) amino acid residue comprises a serum albumin binding side chain.

In a preferred embodiment, the NMU analog peptide according to the invention comprises a polypeptide having at least 80% sequence identity with the peptide of SEQ ID NO: 6

$X_1X_2X_3X_4X_5X_6X_7X_8X_9X_{10}X_{11}X_{12}S_{13}Q_{14}S_{15}R_{16}G_{17}$YFLFRPRN (SEQ ID NO: 6)

wherein each X may be absent or represent a non-serum albumin binding amino acid residue, characterised in that the agonist peptide comprises an alteration at one of the positions corresponding to positions S13, Q14, S15, R16 and G17 of the peptide of SEQ ID NO: 6, wherein the alteration is a substitution of an amino acid residue with an amino acid residue having a serum albumin binding side chain or an insertion of an amino acid having a serum albumin binding side chain.

A highly preferred NMU analog peptide according to the invention comprises a polypeptide having the sequence

$X_1X_2X_3X_4X_5X_6X_7X_8X_9X_{10}X_{11}X_{12}K_{13}Q_{14}S_{15}$RGYFLFRPRN (SEQ ID NO: 7)

wherein each X may be absent or represent a non-serum albumin binding amino acid, characterised in that the lysine$_{13}$ ($K_{13}$) amino acid residue comprises a serum albumin binding side chain.

A highly preferred NMU analog peptide according to the invention comprises a polypeptide having the sequence

FRVDEEFQSPFAKQSRGYFLFRPRN (SEQ ID NO: 8)

characterised in that the lysine$_{13}$ ($K_{13}$) amino acid residue comprises a serum albumin binding side chain.

In a preferred embodiment, the NMU analog peptide according to the invention comprises a polypeptide having at least 80% sequence identity with the peptide of SEQ ID NO: 9

$X_1X_2X_3X_4X_5X_6X_7X_8S_9P_{10}F_{11}A_{12}S_{13}Q_{14}S_{15}R_{16}G_{17}$YFLFRPRN (SEQ ID NO: 9)

wherein each X may be absent or represent a non-serum albumin binding amino acid, characterised in that the agonist peptide comprises an alteration at one of the positions corresponding to positions S9, P10, F11, A12, S13, Q14, S15, R16 and G17 of the peptide of SEQ ID NO: 9, wherein the alteration is a substitution of an amino acid residue with an amino acid residue having a serum albumin binding side chain or an insertion of an amino acid having a serum albumin binding side chain.

A highly preferred NMU analog peptide according to the invention comprises a polypeptide having the sequence

$X_1X_2X_3X_4X_5X_6X_7X_8K_9P_{10}F_{11}A_{12}S_{13}Q_{14}S_{15}$RGYFLFRPRN (SEQ ID NO: 10)

wherein each X may be absent or represent a non-serum albumin binding amino acid, characterised in that the lysine$_9$ ($K_9$) amino acid residue comprises a serum albumin binding side chain.

A highly preferred NMU analog peptide according to the invention comprises a polypeptide having the sequence

FRVDEEFQKPFASQSRGYFLFRPRN (SEQ ID NO: 11)

characterised in that the lysine$_9$ ($K_9$) amino acid residue comprises a serum albumin binding side chain.

In a preferred embodiment, the NMU analog peptide according to the invention comprises a polypeptide having at least 80% sequence identity with the peptide of SEQ ID NO: 12

$X_1X_2X_3X_4X_5E_6F_7Q_8S_9P_{10}F_{11}A_{12}S_{13}Q_{14}S_{15}R_{16}G_{17}$YFLFRPRN (SEQ ID NO: 12)

wherein each X may be absent or represent a non-serum albumin binding amino acid, characterised in that the agonist peptide comprises an alteration at one of the positions corresponding to positions E6, F7, Q8, S9, P10, F11, A12, S13, Q14, S15, R16 and G17 of the peptide of SEQ ID NO: 12, wherein the alteration is a substitution of an amino acid residue with an amino acid residue having a serum albumin binding side chain or an insertion of an amino acid having a serum albumin binding side chain.

A highly preferred NMU analog peptide according to the invention comprises a polypeptide having the sequence

$X_1X_2X_3X_4X_5K_6F_7Q_8S_9P_{10}F_{11}A_{12}S_{13}Q_{14}S_{15}$RGYFLFRPRN (SEQ ID NO: 13)

wherein each X may be absent or represent a non-serum albumin binding amino acid, characterised in that the lysine$_6$

($K_6$) amino acid residue comprises a serum albumin binding side chain.

A highly preferred NMU analog peptide according to the invention comprises a polypeptide having the sequence

FRVDEKFQSPFASQSRGYFLFRPRN (SEQ ID NO: 14)

characterised in that the lysine$_6$ ($K_6$) amino acid residue comprises a serum albumin binding side chain.

In a preferred embodiment, the NMU analog peptide according to the invention comprises a polypeptide having at least 80% sequence identity with the peptide of SEQ ID NO: 15

$X_1X_2X_3D_4E_5E_6F_7Q_8S_9P_{10}F_{11}A_{12}S_{13}Q_{14}S_{15}R_{16}G_{17}$YFLFRPRN (SEQ ID NO: 15)

wherein each X may be absent or represent a non-serum albumin binding amino acid, characterised in that the agonist peptide comprises an alteration at one of the positions corresponding to positions D4, E5, E6, F7, Q8, S9, P10, F11, A12, S13, Q14, S15, R16 and G17 of the peptide of SEQ ID NO: 15, wherein the alteration is a substitution of an amino acid residue with an amino acid residue having a serum albumin binding side chain or an insertion of an amino acid having a serum albumin binding side chain.

A highly preferred NMU analog peptide according to the invention comprises a polypeptide having the sequence

$X_1X_2X_3K_4E_5E_6F_7Q_8S_9P_{10}F_{11}A_{12}S_{13}Q_{14}S_{15}$RGYFLFRPRN (SEQ ID NO: 16)

wherein each X may be absent or represent a non-serum albumin binding amino acid, characterised in that the lysine$_4$ ($K_4$) amino acid residue comprises a serum albumin binding side chain.

A highly preferred NMU analog peptide according to the invention comprises a polypeptide having the sequence

FRVKEEFQSPFASQSRGYFLFRPRN (SEQ ID NO: 17)

characterised in that the lysine$_4$ ($K_4$) amino acid residue comprises a serum albumin binding side chain.

In a highly preferred embodiment of the invention, the peptide according to the invention comprises a sequence selected among SEQ ID NO: 3, 9 and 15. In a more highly preferred embodiment of the invention, the peptide according to the invention comprises a sequence selected among SEQ ID NO: 4, 10 and 16. In an even more highly preferred embodiment of the invention, the peptide according to the invention comprises a sequence selected among SEQ ID NO: 5, 11 and 17.

In a preferred embodiment the serum albumin binding amino acid residue is distanced by less than 14 amino acid residues, such as less than 13 amino acid residues, such as less than 12 amino acid residues, such as less than 11 amino acid residues, such as less than 10 amino acid residues from the first part of the peptide, but at a distance of at least 2 amino acid residues from the first part of the peptide.

In another preferred embodiment the serum albumin binding amino acid residue is distanced by less than 9 amino acid residues, such as less than 8 amino acid residues, such as less than 7 amino acid residues, such as less than 6 amino acid residues, such as less than 5 amino acid residues, such as less than 4 amino acid residues, such as less than 3 amino acid residues from the first part of the peptide, but at a distance of at least 2 amino acid residues from the first part of the peptide.

Serum albumin binding amino acids:

In one embodiment the serum albumin binding side chain is attached to the N-terminal amino group or the side chain of an amino acid residue, such as the side chain of lysine (i.e. ε-N-alkylated lysine), 2,3-diaminopropionic acid, 2,4-diaminobutyric acid, ornithine, O-aminopropylserine or longer O-alkylated serines containing a primary amino group.

Preferably, the serum albumin binding amino acid residue is inserted as a substitution into e.g. the sequence of SEQ ID NO: 2.

The serum albumin binding amino acid residue comprises a serum albumin binding side chain. Preferably, serum albumin binding side chains according to the present invention are side chains comprising a lipid molecule. In broad terms the amino acid residues comprising a serum albumin binding side chain according to the present invention are lipidated amino acid residues.

In one embodiment the serum albumin binding side chain comprises an alkyl chain with at least 14 carbon atoms, wherein said alkyl chain comprises a distal carboxylic acid or a distal tetrazole group and wherein said alkyl chain comprises a proximal carbonyl group.

In one embodiment the serum albumin binding side chain comprises the structure A, wherein A is selected among

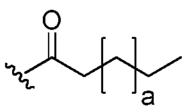
,
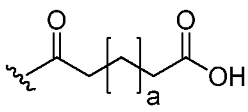
or
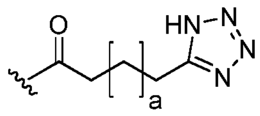

where a is at least 10.

In a preferred embodiment of the present invention, the serum albumin binding side chain is selected from the group consisting of A-B-C-, A-B- and A-C-, wherein A is

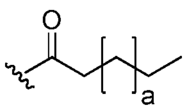
,
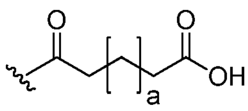
or
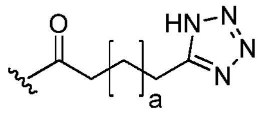

where a is selected from the group consisting of 10, 11, 12, 14, 15, 16, 17 and 18 and

B is selected from

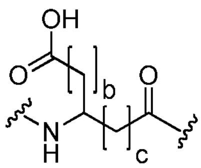
,
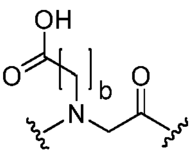
or
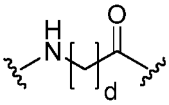

Wherein b is selected from the group consisting of 0, 1, and 2, c is selected from the group consisting of 0, 1 and 2, and d is selected from the group consisting of 1, 2, 3, 4, 5, 6, 7, 8, 9, 10, 11 and 12.

C is a spacer molecule, such as at least one D, E, S, T, K, R, G, A, Ornithine, Citrulline, 8-amino-3,6-dioxaoctanoic acid or 12-amino-4,7,10-trioxaoctadecanoic acid molecule.

Serum albumin binding side chains according to the present invention are described e.g. in WO2011058165.

An example of A-B-C- is:

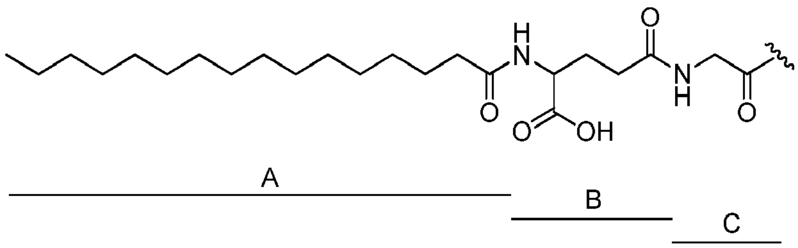

An example of A-B- is:

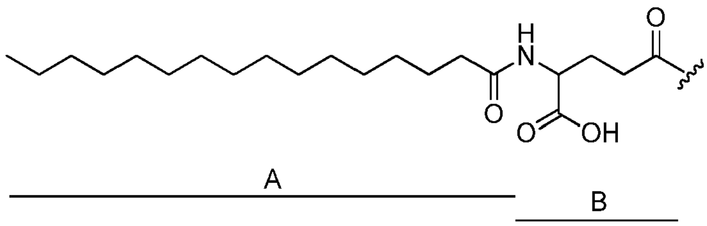

An example of A-C- is:

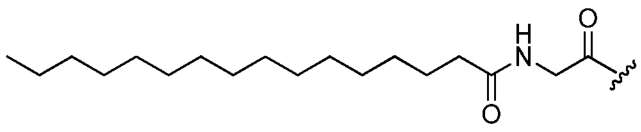

A

C

In one embodiment the serum albumin binding side chain comprises at least one dicarboxylic acid, such as hexadecanedioic acid, octadecanedioic acid or dodecanedioic acid.

It is believed that the negative charge in the distal end of the dicarboxylic acid increases affinity of the NMU derivative to serum albumin. In one embodiment the fatty di-acid or tetrazole may be attached to a spacer, such as a negatively charged amino acid, e.g., L-gamma-glutamate. In one embodiment the alkyl chain, optionally comprising a dicarboxylic acid or a tetrazole group, may be attached to a spacer. In one embodiment the combined alkyl chain, optionally comprising a dicarboxylic acid or a tetrazole group, and the negatively charged amino acid may be separated with a spacer.

The peptides according to the invention are useful for treatment or prophylactic treatment of a human or animal subject. Thus, in a preferred embodiment of the invention the peptides according to the invention as described above are for treatment or prophylactic treatment of a human or animal subject.

Preferably, the animal subject is a vertebrate. Even more preferably, the animal subject is a mammal.

The preferred treatment is a treatment of a metabolic disease. One preferred metabolic disease for treatment according to the present invention is obesity. Another preferred metabolic disease for treatment according to the present invention is diabetes.

Accordingly, in embodiment, the present invention relates to the use of the peptides according to the invention as described above for treatment or prophylactic treatment of metabolic disease in a human or animal subject.

In another embodiment, the present invention relates to the use of the peptides according to the invention as described above for treatment or prophylactic treatment of obesity in a human or animal subject.

In another embodiment, the present invention relates to the use of the peptides according to the invention as described above for treatment or prophylactic treatment of diabetes in a human or animal subject.

The present invention also relates to pharmaceutical or veterinary composition comprising a peptide according to the invention as described above and at least one pharmaceutical or veterinary excipient. The invention also relates to the use of a peptide according to the invention as described above or a composition comprising a peptide according to the invention as described above for medical or veterinary treatment of a human or animal subject, preferably the treatment of metabolic disorders such as obesity and diabetes.

The skilled person is well aware of how to produce pharmaceutical compositions in general.

Preferably the compositions according to the invention are in unit dosage forms providing a daily dose of a therapeutically effective amount of peptide according to the invention.

Preferably, the unit dosage form is a tablet.

The invention also relates to a method of treatment of a human or animal subject, the method comprising administering a peptide according to the invention as described above to a human or animal subject.

## Examples

*Materials and methods*

*Peptide Synthesis*

NMU peptide derivatives or analogues thereof of the invention may be synthesized by standard solid-phase peptide synthesis (SPPS) using either an automated peptide synthesizer, or traditional bench synthesis. All reagents and solvents were purchased at Iris Pharmaceuticals GmbH or Sigma-Aldrich, except the TentaGel S Rink amide resin which was purchased from RAPP Polymere GmbH.

Solid-phase peptide synthesis: The peptides were synthesized on TentaGel S Rink Amide (0.24-0.26 mmol/g) resin using the Fmoc/tBu (Fluoren-9-ylmethyloxycarbonyl / tert-butyl) strategy by automated solid-phase peptide synthesis (SPPS) on a MultiSynTech Syro II peptiede synthesizer. The resin was swelled first in DCM for 30 min and then in NMP for 2 min. The $N^{\alpha}$-Fmoc-protected amino acids (4 eq.) were coupled at room temperature using N,N'-diisopropylcarbodiimide (DIC) (4 eq.) and ethyl 2-cyano-2-(hydroxyimino)acetate (Oxyma) (4 eq.) in N,N-dimethylformamide (DMF). Coupling times were 2 times 2 hr. The $N^{\alpha}$-deprotections were performed using first 40% piperidine in N-methyl-2-pyrrolidinone (NMP) for 3 min followed by 20% piperidine in NMP for 22 min. The resin was washed in-between each reaction by 2 times NMP for 1 min, 1 time dichloromethane (DCM) for 1 min and 2 times NMP for 1 min. The peptides were either site-specific lipidated by pH-controlled acylation in solution or by on-resin acylation using orthogonal protecting groups. For the on-resin lipidation a tert-butyloxycarbonyl ($N^{\alpha}$-Boc) protected amino acid was used and the lipidation site was side-chain protected during peptide elongation using with allyloxycarbonyl (Alloc). The SPPS was continued in a stepwise manner until the desired sequence was obtained. At the end the peptide was either acylated on-resin or released from the solid support and simultaneously side-chain deprotected followed by solution phase acylation.

Solution phase acylation: The peptides were side-chain deprotected and simultaneously cleaved from the solid support by a trifluoroacetic acid (TFA) cocktail containing triethyl silane (TES) and water (95/3/2) as scavengers. The crude peptides were purified by HPLC using reverse phase C18 Gemini column (5μm, 110 Å, 100 mm x 21.2 mm) and water/0.1% TFA (solvent A) acetonitrile/0.1% TFA (solvent B) as the mobile phases. A linear gradient from 10 to 50% of solvent B (0-27 min) at 15 mL/min was applied on a Dionex Ultimate 3000 semiprepative HPLC system. The purified peptides were characterized using LC/MS-ESI (MSQ Plus Mass spectrometer (Thermo Fisher Scientific, INc.), Dionex Ulitimate 3000 HPLC system equipped with a PDA UV detector). The lipidation reaction was performed by coupling the $N^{\alpha}$-hexadecanoyl-L-γ-Glu(ONHS)-OtBu (1.5 eq), in solution, to the epsilon amine of the free lysine in sodium carbonate buffer at pH 10.5-11. The peptides were dissolved in 2 mg/mL in 0.1 M sodium carbonate ($Na_2CO_3$) and the lipid was dissolved in small amount of acetonitrile - mixed and stirred at room temperature for 4 hours. The peptide mixtures were freeze dried and side-chain deprotected by addition of 95% TFA, 3% TES, 2% water for 2 hr. The TFA mixture was concentrated by nitrogen flow and re-dissolved in water/acetonitrile (50/50) for preparative HPLC purification. The lipidated peptides were purified by preparative HPLC using reverse phase as described above. The purified peptides were characterized using LC/MS-ESI as described above.

On-resin acylation: The site of acylation was carried out by deprotecting the $N^{\varepsilon}$-Allyloxycarbonyl-L-lysine (Lys(Alloc)) using 1.0 eq. of tetrakis(triphenylphosphine)-palladium(0) ($Pd(PPh_3)_4$) and 1.5 eq. of borane dimethyl amine complex for 1 hour at room temperature and then subsequently coupling $N^{\alpha}$-(9-fluorenylmethyloxycarbonyl)-L-glutamic acid α-tert-butyl ester (Fmoc-L-Glu-OtBu) using N-[(dimethylamino)-1H-1,2,3-triazole[4,5-b]pyridine-1-ylmethylene]-N-methylmethanaminium hexafluorophosphate N-oxide (HATU) (4 eq.) and N,N-diisopropylethylamine (DIEA) (7.2 eq.) for 15 hours. The Fmoc-group was removed by 40% piperidine in NMP for 3 min followed by 20% piperidine in NMP for 22 min. Finally, the palmitic acid was coupled using HATU (4 eq.) and DIEA (7.2 eq.) for 2 times 2 hours. The peptide mixtures were freeze dried and side-chain deprotected by addition of 95% TFA, 3% TES, 2% water for 2 hr. The lipidated peptides were purified by preparative HPLC as described above. The purified peptides were characterized using LC/MS-ESI as described above.

| **Molecular weight calculated and found by ESI MS** | | | | | |
|---|---|---|---|---|---|
| Compound | Acylation position | MS Calc. | | MS found | |
| | | $[M+2H]^{2+}$ | $[M+3H]^{3+}$ | $[M+2H]^{2+}$ | $[M+3H]^{3+}$ |
| Human NMU | None | 1541.0 | 1027.7 | 1540.5 | 1027.5 |
| SEQ ID NO 5 | K15 | 1730.9 | 1154.3 | 1730.1 | 1154.0 |
| SEQ ID NO 8 | K13 | 1724.4 | 1150.0 | 1722.7 | 1148.7 |
| SEQ ID NO 11 | K9 | 1745.4 | 1164.0 | 1744.3 | 1163.3 |
| SEQ ID NO 14 | K6 | 1745.4 | 1164.0 | 1744.3 | 1162.6 |
| SEQ ID NO 17 | K4 | 1745.4 | 1164.0 | 1744.2 | 1162.6 |

*Functional assay*

All compounds were dissolved in dimethyl sulfoxide (DMSO), and then water was added (total DMSO conc. was 80%). The stock concentration was 1 mM for all the compounds. Stocks were alliquoted and frozen -20°C.

The day before each assay a new standard row was prepared by dilution of the stock solution in assay buffer (1 mM acetic acid + 0.1% bovine serum albumin (BSA)). The following concentrations were prepared (depending on the ligand potency): $10^{-5}$, $10^{-6}$, $10^{-7}$, $10^{-7.5}$, $10^{-8}$, $10^{-8.5}$, $10^{-9}$, $10^{-10}$, $10^{-11}$, $10^{-12}$ M.

Cell cultures: The HEK293 cells are grown in (DMEM31966 glutamax supplemented with 10% FBS (fetal bovine serum) and 1% penicillin G and streptomycin) and incubated at 37°C with 10% $CO_2$ in a humidified atmosphere. The cells were split as follows. HEK293 cells approximately 80% confluent was used. The cells were removed from the bottom of the bottle by removing the media (DMEM31966 glutamax supplemented with 10% FBS and 1% penicillin G

and streptomycin), rinsed with 5 mL phosphate buffered saline (PBS) and shaken gently. The PBS was removed and cells were released from the bottom with trypsin (1-2 mL) and incubated for 2-3 min. The flasks were then turned gently to ensure complete release of the cells from the bottom. Then media was added to the cells, (5-8 mL was used to rinse the cells of the bottom, this process was repeated approximately 4 times using the same media). Cell suspension was then transferred to a new bottle (approximately 4 × 106 cells per 175 $cm^2$ flask) and 20 mL media was added. Cells were incubated at 37°C with 10% $CO_2$ in a humidified atmosphere.

Transfection of cells: HEK293 cells were seeded out at the density of 8 × $10^6$ cells per 175 $cm^2$ flask 1 day prior to the transfection. Before transfection of cells, the culture medium was aspirated and half of the standard volume was added. 40 μg receptor-containing plasmid (Neuromedin U receptor 1 (NMUR1) or Neuromedin U receptor 2 (NMUR2)) or "empty" vector plasmid (pcDNA3), as a negative control, was mixed with 60 μL 2 mM $CaCl_2$ and Tris-EDTA (TE) buffer (10 mM tris(hydroxymethyl)aminomethane hydrochloride (Tris HCl), 1 mM ethylenedinitrilotetraacetic acid (EDTA), pH 7.5) to a total volume of 480 μL. The mixture was added drop-wise to another tube containing 480 μL 2 × HEPES buffered saline (HBS buffer) (280 mM NaCl, 50 mM 4-(2-Hydroxyethyl)piperazine-1-ethanesulfonic acid (HEPES), 1.5 mM $NaHPO_4$, pH 7.2) and left for 45 min at room temperature for DNA precipitation. Afterwards, the precipitate was added carefully to the cells and cells were incubated for 5 hours at normal culture conditions. The transfection was stopped by change of the DNA-containing medium with 20 mL of fresh cell culture medium.

Seeding of the cells in 96 well plates: One day after transfection, cells were harvested using trypsin as described above. The cells were seeded in a poly-D-lysine coated clear 96 well plate. 100 μL cell suspension 350.000 cells/mL was added. 3H-myo-inositol (5μL/mL) was added to each well yielding a concentration of 35000 cells/well. The cells were incubated overnight before assay procedure the following day.

$IP_3$ SPA-ysi assay: The media was removed and the plates were washed with 200 μL of Hank's balanced salt solution (HBSS) (Gibco, Life Technologies). 100 μL of HBSS/10 mM LiCl was added and the plates were incubated 15 min at 37°C. 5 μL ligand was added to each well and the plate was incubated at 37°C for 70-90 min (incubation time was approximately 1 h and 16 min for all plates). The plates were placed on ice and 50 μL formic acid (10 mM) was added to each well. The plates was left on ice for at least 30 min. 20 μL was then transferred to a white 96 well plate and 80 μL yttrium silicate (YSi) SPA-beads RPNQ0010 (Perkin Elmer) solution (0.0125 g/mL) is added to each well. The plates were sealed and shaken on high speed for 30 min, then centrifuged for 5 min at 1500 rpm and counted with 8 hours delay in the top counter.

Calculation of $EC_{50}$ values: The potency of the analogues was determined via the $EC_{50}$ values. The $EC_{50}$ value is dependent on the analogues ability to bind (affinity) and activate (efficacy) the receptor and is defined as the agonist concentration, which produces half maximal effect. The $EC_{50}$ value is calculated by the Hill equation: $E = (E_{max} * C^n)/(EC^n{}_{50} + C^n)$, where C is the agonist concentration, $E_{max}$ is the maximal effect, and n is the Hill coefficient.

Data, reporting, and statistical evaluation: All data were fed into Excel 2003 spread sheets. Statistics and data analyses were performed using Graph Pad Prism 4.0 software. Curve fitting were performed by using GraphPad PRISM sigmoidal dose-response (variable slope). The equation for sigmoidal dose-response (variable slope) is Y = Bottom + (Top-Bottom)/(1+10^((Log$EC_{50}$-X)*HillSlope)). Results are presented as mean±SEM (standard error of the mean) unless otherwise stated. n denotes the number of times a ligand were tested in the assay. All assay measurements were performed in duplicates.

*In vivo studies.*

Animals: A total of seventy two (72) male NMRI mice 6-7 weeks old (approx. 25 g BW) at the time of arrival were obtained from Taconic (Denmark) and transferred to the Gubra test stables. Upon arrival the animals will be transferred into the HM2 cages and housed in groups of four. The animal room environment were controlled (targeted ranges: temperature 22 ± 2°C; relative humidity 50 ± 10%; light/dark cycle: 12 hours light, 12 hours dark, lights on from 6 AM to 6 PM. Rats have ad libitum access to Altromin (Brogaarden, Denmark) and tap water.

All animal experiments were conducted in accordance with Gubra ApS bioethical guidelines, which are in full compliance with internationally accepted principles for the care and use of laboratory animals. The described experiments are covered by personal licenses to Jacob Jelsing (2013-15-2934-00784) issued by the Danish Committee for animal research.

Run-in period: Mice arrived at day -7. A minimum of 5-7 days of habituation to the system was allowed before study start. During these days, animals were handled daily to accustom them to the experimental paradigm.

Runs in the HM2 system: In total five runs were performed in the HM2 system. There were at least 4 days of washout between each run.

Pilot study (PD study): Before the acute *in vivo* study was initiated a pilot study will be performed with the aim to identify a dose of NMU, which has acute effect on food intake. NMU was tested in 3 different doses: 1 mg/kg, 3 mg/kg and 10 mg/kg (n=4). In addition, PYY3-36 in 1 mg/kg and vehicle were tested. Upon completion of the pilot study, the mice will be re-randomized to enter the full study (see below).

Acute effect of lipidated analogues: Mice were re-randomized and the acute effects of the lipidated compounds were tested in an acute setting. Mice were dosed in doses equimolar to the concentration of NMU found to be semi-efficacious.

Dose response study: Different doses of the analogues were tested in order to establish a dose response relationship. Mice were re-randomized and four different doses of the five lipidated analogues were tested over two different runs in the HM2 system and compared with NMU at 1 pmol/kg and liraglutide at 0.05 μmol/kg.

Randomization and experimentation: Animals were randomized according to body weight the day of dosing and stratified into the following experimental groups based on mean cage weight (n=4-8):

Pilot (PD) study run 1 (dose finding of NMU):

1: Vehicle (0.9% NaCl, pH ~ 4.0 and 0.1% BSA) (n=4)
2: Native human Neuromedin U (NMU-25), sc, 1 mg/kg (n=4)
3: Native human Neuromedin U (NMU-25), sc, 3 mg/kg (n=4)
4: Native human Neuromedin U (NMU-25), sc, 10 mg/kg (n=4)
5: PYY3-36, sc 1 mg/kg (n=4)

Pilot (PD) study run 2 (dose finding of NMU):

1: Vehicle (0.9% NaCl, pH ~ 4.0 and 0.1% BSA) (n=4)
2: Native human Neuromedin U (NMU-25), sc, 1 mg/kg (n=4)
3: Native human Neuromedin U (NMU-25), sc, 3 mg/kg (n=4)

Acute effects of lipidated analogues:

1: Vehicle (PBS + 0.1% BSA+ 5.4% DMSO) SC
2: Liraglutide 0.2 mg/kg, sc, 0.05 pmol/kg (n=8)
3: Human neuromedin U 25 (NMU), sc, 1 μmol/kg (n=8)
5: SEQ ID NO 17, sc, 1 pmol/kg (n=8)
6: SEQ ID NO 14, sc, 1 pmol/kg (n=8)
7: SEQ ID NO 11, sc, 1 pmol/kg (n=8)
8: SEQ ID NO 8, sc, 1 pmol/kg (n=8)
9: SEQ ID NO 5, sc, 1 pmol/kg (n=8)

Dose response study run 1

1: Vehicle (0.9% NaCl, pH ~ 4.1 and 0.1% BSA) (n=6)
2: Liraglutide 0.2 mg/kg, sc, 0.05 pmol/kg (n=4)
3: Human neuromedin U 25 (NMU), sc, 1 μmol/kg (n=5)
8: SEQ ID NO 17, sc, 1 pmol/kg (n=4)
9: SEQ ID NO 17, sc, 0.3 pmol/kg (n=5)
10: SEQ ID NO 17, sc, 0.1 μmol/kg (n=5)
11: SEQ ID NO 17, sc, 0.03 pmol/kg (n=5)
12: SEQ ID NO 14, sc, 1 pmol/kg (n=4)
13: SEQ ID NO 14, sc, 0.3 pmol/kg (n=5)
14: SEQ ID NO 14, sc, 0.1 pmol/kg (n=5)
15: SEQ ID NO 14, sc, 0.03 pmol/kg (n=5)

Dose response study run 2

1: Vehicle (0.9%NaCl, pH ~ 4.0 and 0.1% BSA) (n=5)
2: Liraglutide 0.2 mg/kg, sc, 0.05 pmol/kg (n=4)
3: Human neuromedin U 25 (NMU), sc, 1 μmol/kg (n=4)
4: SEQ ID NO 11, sc, 1 pmol/kg (n=4)
5: SEQ ID NO 11, sc, 0.3 pmol/kg (n=5)
6: SEQ ID NO 11, sc, 0.1 pmol/kg (n=5)
7: SEQ ID NO 11, sc, 0.03 pmol/kg (n=5)
8: SEQ ID NO 8, sc, 1 pmol/kg (n=4)

9: SEQ ID NO 8, sc, 0.3 pmol/kg (n=5)
10: SEQ ID NO 8, sc, 0.1 pmol/kg (n=5)
11: SEQ ID NO 8, sc, 0.03 pmol/kg (n=5)
12: SEQ ID NO 5, sc, 1 pmol/kg (n=4)
13: SEQ ID NO 5, sc, 0.3 pmol/kg (n=5)
14: SEQ ID NO 5, sc, 0.1 μmol/kg (n=5)
15: SEQ ID NO 5, sc, 0.03 pmol/kg (n=5)

Dosing: All dosing took place just prior to lights out in the afternoon. Doses was administered subcutaneously (sc) (dose volume 10 ml/kg), alternating the injection site from the left to the right side of the lower back and inguinal region, respectively.
Body weight, food- and water intake: Food intake data was collected by advanced synchronous real-time monitoring of food intake behaviour in the HM2 system. Food and water intake were measured from day -1 and for 24 hours after each dose. Body weight was measured from day -7 and throughout the study.
Health status observations: Cage-side signs of illness (ill health, behavioural changes etc.) were observed by the animal technicians during the pre-treatment and treatment period immediately before and after dosing.
Identification: Animals were uniquely identified with implantable microchips (Pet ID Microchip, E-vet), which were implanted under light $CO_2$ anaesthesia in all rats upon arrival. Animals were identified using the WS-1 weigh station (MBrose ApS, Faaborg, Denmark) connected to a laptop running the HM02Lab software (Ellegaard Systems, Faaborg, Denmark). The HM2 Lab software matches body weight with ID and calculates dose directly based on the body weight.
Statistical Evaluation: All data are fed into Excel 5.0 spread sheets and subsequently subjected to relevant statistical analyses (Graph Pad software). Results are presented as mean±SEM (standard error of the mean) unless otherwise stated. Statistical evaluation of the data is carried out using one-way analysis of variance (ANOVA) with appropriate post-hoc analysis between control and treatment groups in cases where statistical significance is established ($p<0.05$; Bonferoni).

***Results***

Example 1

| **The activity of NMU analogues in NMUR1 and NMUR2 receptor assays as a function of acylation position** | | | | | |
|---|---|---|---|---|---|
| Compound | Acylation position | NMUR1 cAMP $EC_{50}$ (nM) | | NMUR2 cAMP $EC_{50}$ (nM) | |
| Human NMU | None | 2.57 | | 4.26 | |
| SEQ ID NO 5 | K15 | 194.69 | | 162.44 | |
| SEQ ID NO 8 | K13 | 114.10 | | 154.66 | |
| SEQ ID NO 11 | K9 | 74.02 | | 94.26 | |
| SEQ ID NO 14 | K6 | 51.45 | | 52.78 | |
| SEQ ID NO 17 | K4 | 45.47 | | 50.46 | |
| | | | | | |
| **Percent food intake reduction 6 hours post SC dosing** | | | | | |
| Compound | Acylation position | μmol/kg | | | |
| | | 1.0 | 0.3 | 0.1 | 0.03 |
| Human NMU | None | 25 | | | |
| SEQ ID NO 5 | K15 | 76 | 61 | 41 | 41 |
| SEQ ID NO 8 | K13 | 90 | 46 | 55 | 25 |
| SEQ ID NO 11 | K9 | 85 | 78 | 56 | 46 |
| SEQ ID NO 14 | K6 | 89 | 79 | 31 | 5 |

(continued)

| Percent food intake reduction 6 hours post SC dosing | | | | | |
|---|---|---|---|---|---|
| Compound | Acylation position | μmol/kg | | | |
| | | 1.0 | 0.3 | 0.1 | 0.03 |
| SEQ ID NO 17 | K4 | 78 | 64 | 43 | 20 |

## Claims

1. A Neuromedin U receptor agonist peptide comprising a first C-terminal part and a second N-terminal part, said first part comprising an amino acid sequence corresponding to the sequence of SEQ ID NO: 1;
YFLFRPRN (SEQ ID NO: 1) or a functional analog thereof,
said second part comprising at least two amino acid residues wherein at least one amino acid residue is a serum albumin binding amino acid residue, said serum albumin binding amino acid residue being distanced by at least one but less than 16 amino acids from the first part of the peptide.

2. Peptide according to claim 1, wherein the first part amino acid sequence, independently of the second part, corresponds to a peptide capable of binding one or of NMU receptors NMUR1 and/or NMUR2 at an $EC_{50}$ value of less than 1000 nM.

3. Peptide according to claim 1 or 2, wherein the first part amino acid sequence has at least 60% sequence identity, such as at least 70%, such as at least 80% sequence identity with the peptide of SEQ ID NO: 1.

4. Peptide according to any of claims 1-3, wherein the first part and the second part together correspond to a polypeptide having at least 70% sequence identity with the peptide of SEQ ID NO: 2;
$X_1X_2X_3X_4X_5X_6X_7X_8X_9X_{10}X_{11}X_{12}X_{13}X_{14}X_{15}X_{16}X_{17}$YFLFRPRN (SEQ ID NO: 2)
wherein each X may be absent or represent a non-serum albumin binding amino acid residue, with the proviso that the second part comprises at least two amino acid residues, **characterised in that** the second part of the peptide comprises at least one alteration at any one of the positions corresponding to positions $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $X_{15}$ or $X_{16}$ of the peptide of SEQ ID NO: 2, wherein the alteration is a substitution of the respective amino acid residue with a serum albumin binding amino acid residue or an insertion of a serum albumin binding amino acid residue.

5. Peptide according to any of claims 1-4, wherein the first part and the second part together correspond to a polypeptide having at least 70% sequence identity with the peptide of SEQ ID NO: 3;
$X_1X_2X_3X_4X_5X_6X_7X_8X_9X_{10}X_{11}X_{12}X_{13}X_{14}S_{15}R_{16}G_{17}$YFLFRPRN (SEQ ID NO: 3)
wherein each X may be absent or represent a non-serum albumin binding amino acid, **characterised in that** the peptide comprises at least one alteration at any one of the positions corresponding to positions $X_1$, $X_2$, $X_3$, $X_4$, $X_5$, $X_6$, $X_7$, $X_8$, $X_9$, $X_{10}$, $X_{11}$, $X_{12}$, $X_{13}$, $X_{14}$, $S_{15}$, $R_{16}$ or $G_{17}$ of the peptide of SEQ ID NO: 3, wherein the alteration is a substitution of the amino acid with a serum albumin binding amino acid or an insertion of a serum albumin binding amino acid.

6. Peptide according to claim 4 or 5, wherein:

a. $X_2$ is selected among R, K, Ornithine, Citrulline, N-methyl arginine, N-methyllysine, N-methyl ornithine, Methyl lysine, Dimethyl lysine, Trimethyl lysine, 2-Amino-3-guanidino-propionic acid, 2-Amino-4-guanidino-butyric acid or Monomethyl arginine, Dimethyl arginine, (2-Guanidino-ethylamino)-acetic acid, (3-Guanidino-propylamino)-acetic acid, (4-Guanidino-butylamino)-acetic acid, 2-Amino-3-(4-guanidino-phenyl)propionic acid, 2,3-Diaminopropionic acid, 2,4-Diaminobutyric acid or absent; and
b. $X_3$ is selected among V, L, I, Aminoisobutyric acid, Homoleucine, Norleucine, 1-Aminobutyric acid, 2,3-Diaminopropionic acid, 2,4-Diaminobutyric acid, Ornithine, K or absent; $X_4$ is selected among D, E, Homoglutamic acid, 2,3-Diaminopropionic acid, 2,4-Diaminobutyric acid, Ornithine, K or absent; and
c. $X_5$ is selected among E, D, Homoglutamic acid, 2,3-Diaminopropionic acid, 2,4-Diaminobutyric acid, Ornithine, K or absent; and
d. $X_6$ is selected among E, D, Homoglutamic acid, 2,3-Diaminopropionic acid, 2,4-Diaminobutyric acid, Ornithine, K or absent; and
e. $X_7$ is selected among F, Y, Homophenylalanine, Homotyrosine, Phenylglycine, N-methyl Phenylalanine, N-methyl Tyrosine, 2,3-Diaminopropionic acid, 2,4-Diaminobutyric acid, Ornithine, K or absent; and
f. $X_8$ is selected among Q, N, N-methyl Glutamine, N-methyl Asparagine, 2,3-Diaminopropionic acid, 2,4-Diaminobutyric acid, Ornithine, K or absent; and
g. $X_9$ is selected among S, T, N-methyl Serine, N-methyl Threonine, Homoserine, Homothreonine, O-methyl

Threonine, O-methyl Serine, 2,3-Diaminopropionic acid, 2,4-Diaminobutyric acid, Ornithine, K or absent; and
h. $X_{10}$ is selected among P, Hydroxyproline, 2,3-Diaminopropionic acid, 2,4-Diaminobutyric acid, Ornithine, K or absent; and
i. $X_{11}$, is selected among F, Y, Homo Phenylalanine, Homo Tyrosine, Phenylglycine, N-methyl Phenylalanine, N-methyl Tyrosine, 2,3-Diaminopropionic acid, 2,4-Diaminobutyric acid, Ornithine, K or absent; and
j. $X_{12}$ is selected among A, Aib, N-methyl Alanine 2,3-Diaminopropionic acid, 2,4-Diaminobutyric acid, Ornithine, K or absent; and
k. $X_{13}$ is selected among S, T, N-methyl Serine, N-methyl Threonine, Homoserine, Homothreonine, O-methyl Threonine, O-methyl Serine, 2,3-Diaminopropionic acid, 2,4-Diaminobutyric acid, Ornithine, K or absent; and
l. $X_{14}$ is selected among Q, N, N-methyl Glutamine, N-methyl Asparagine, 2,3-Diaminopropionic acid, 2,4-Diaminobutyric acid, Ornithine, K or absent.

**7.** Peptide according to any of claims 4 - 6, wherein $X_2$ is R or absent, $X_3$ is V or absent, $X_4$ is D or absent, $X_5$ is E or absent, $X_6$ is E or absent, $X_7$ is F or absent, $X_8$ is Q or absent, $X_9$ is S or absent, $X_{10}$ is P or absent, $X_{11}$, is F or absent, $X_{12}$ is A or absent, $X_{13}$ is S or absent and $X_{14}$ is Q or absent.

**8.** Peptide according to any of the proceeding claims, wherein the serum albumin binding amino acid is distanced by less than 14, such as less than 9, amino acid residues from the first part of the peptide.

**9.** Peptide according to any of the proceeding claims, wherein the peptide comprises a sequence having at least 80% sequence identity with the peptide of SEQ ID NO: 11.

**10.** Peptide according to any of the proceeding claims, wherein the peptide comprises a sequence having at least 80% sequence identity with the peptide of SEQ ID NO: 5.

**11.** Peptide according to any of the proceeding claims, wherein the serum albumin binding amino acid is at one of the positions corresponding to positions S9, S13 and S17 of the peptide of SEQ ID NO: 3.

**12.** Peptide according to any of the proceeding claims, wherein said serum albumin binding side chain comprises the structure A, where A is selected among

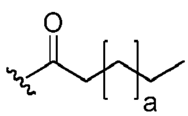 , 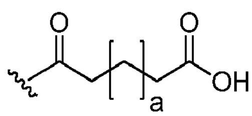 or 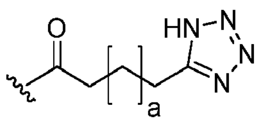

and a is at least 10.

**13.** Peptide according to any of claims 1 - 12 for treatment or prophylactic treatment of a human or animal subject.

**14.** Pharmaceutical or veterinary composition comprising a peptide according to any of claims 1- 13 and at least one pharmaceutical or veterinary excipient.

**15.** Method of treatment of a human or animal subject, the method comprising administering a peptide according to any of claims 1 - 13 to a human or animal subject.

Europäisches Patentamt
European Patent Office
Office européen des brevets

# EUROPEAN SEARCH REPORT

Application Number
EP 13 18 2749

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/312157 A1 (LEVY ODILE ESTHER [US] ET AL) 18 December 2008 (2008-12-18) * sequences 96-98, 459-461 * | 1-15 | INV. C07K14/575 |
| X | US 2006/293232 A1 (LEVY ODILE E [US] ET AL LEVY ODILE ESTHER [US] ET AL) 28 December 2006 (2006-12-28) * paragraphs [0101], [0199] * | 1-15 | |
| X | WO 2010/138343 A1 (MERCK SHARP & DOHME [US]; ANGELETTI P IST RICHERCHE BIO [IT]; PESSI AN) 2 December 2010 (2010-12-02) * pages 14,18; examples 1-8; table 1 * | 1-15 | |
| X,D | US 2012/094898 A1 (ASAMI TAIJI [JP] ET AL) 19 April 2012 (2012-04-19) * pages 15-24 * | 1-15 | |
| X | WO 2009/042053 A2 (MERCK & CO INC [US]; ANGELETTI P IST RICHERCHE BIO [IT]; MARSH DONALD) 2 April 2009 (2009-04-02) * page 15; example 11; table 1 * | 1-15 | TECHNICAL FIELDS SEARCHED (IPC) |
| X | EP 2 418 213 A1 (TAKEDA PHARMACEUTICAL [JP]) 15 February 2012 (2012-02-15) * pages 40-49; examples 2-4 * | 1-15 | C07K |

The present search report has been drawn up for all claims

1

| Place of search | Date of completion of the search | Examiner |
|---|---|---|
| Munich | 18 February 2014 | Pinheiro Vieira, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 18 2749

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2014

| Patent document cited in search report | Publication date | Patent family member(s) | Publication date |
|---|---|---|---|
| US 2008312157 A1 | 18-12-2008 | US 2008312157 A1 | 18-12-2008 |
| | | US 2013137631 A1 | 30-05-2013 |
| US 2006293232 A1 | 28-12-2006 | US 2006293232 A1 | 28-12-2006 |
| | | US 2012253023 A1 | 04-10-2012 |
| WO 2010138343 A1 | 02-12-2010 | NONE | |
| US 2012094898 A1 | 19-04-2012 | AR 083395 A1 | 21-02-2013 |
| | | JP 2013209295 A | 10-10-2013 |
| | | TW 201305219 A | 01-02-2013 |
| | | US 2012094898 A1 | 19-04-2012 |
| | | UY 33665 A | 31-05-2012 |
| | | WO 2012050227 A1 | 19-04-2012 |
| WO 2009042053 A2 | 02-04-2009 | US 2011301079 A1 | 08-12-2011 |
| | | WO 2009042053 A2 | 02-04-2009 |
| EP 2418213 A1 | 15-02-2012 | CA 2758264 A1 | 14-10-2010 |
| | | CN 102459311 A | 16-05-2012 |
| | | EP 2418213 A1 | 15-02-2012 |
| | | US 2012094899 A1 | 19-04-2012 |
| | | WO 2010116752 A1 | 14-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011005611 A1
- WO 2007109135 A2
- US 20120094898 A1
- US 20110275559 A1
- WO 2011058165 A


### Non-patent literature cited in the description


- **BRIGHTON et al.** *Pharmacol. Rev.,* 2004, vol. 56, 231-248
- **FUNES et al.** *Peptides,* 2002, vol. 23, 1607-1615
- *Chem. Pharm Bull.,* 1991, vol. 39, 2319-2322
- *Chem. Pharm. Bull.,* 1995, vol. 43, 1148-1153
- *Chem. Pharm. Bull.,* 1995, vol. 43, 1154-1157
- *Chem. Pharm. Bull.,* 1996, vol. 44, 1180-1884
- *Chem. Pharm. Bull.,* 2000, vol. 48, 1166-1170
- **INGALLINELLA et al.** *Bioorganic & Medicinal Chemistry,* 2010, vol. 20, 4751-4759
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277

SEQUENCE LISTING

```
<110>  Gubra ApS
       Pedersen, Søren Ljungberg

<120>  Neuromedin U analogs comprising serum albumin binding amino acid
       residue

<130>  Lipidated NMU project

<160>  17

<170>  PatentIn version 3.5

<210>  1
<211>  8
<212>  PRT
<213>  Artificial Sequence

<220>
<223>  NMU analog

<400>  1

Tyr Phe Leu Phe Arg Pro Arg Asn
1               5


<210>  2
<211>  25
<212>  PRT
<213>  Artificial Sequence

<220>
<223>  NMU analog


<220>
<221>  misc_feature
<222>   (1)..(17)
<223>  Xaa can be any naturally occurring amino acid

<400>  2

Xaa Xaa Xaa Xaa Xaa Xaa Xaa Xaa Xaa Xaa Xaa Xaa Xaa Xaa Xaa Xaa
1               5                   10                  15


Xaa Tyr Phe Leu Phe Arg Pro Arg Asn
            20                  25


<210>  3
<211>  25
<212>  PRT
<213>  Artificial Sequence

<220>
<223>  NMU analog


<220>
<221>  misc_feature
```

<222> (1)..(14)
<223> Xaa can be any naturally occurring amino acid

<400> 3

```
Xaa Xaa Xaa Xaa Xaa Xaa Xaa Xaa Xaa Xaa Xaa Xaa Xaa Xaa Ser Arg
1               5                   10                  15

Gly Tyr Phe Leu Phe Arg Pro Arg Asn
            20                  25
```

<210> 4
<211> 25
<212> PRT
<213> Artificial Sequence

<220>
<223> NMU analog

<220>
<221> misc_feature
<222> (1)..(14)
<223> Xaa can be any naturally occurring amino acid

<400> 4

```
Xaa Xaa Xaa Xaa Xaa Xaa Xaa Xaa Xaa Xaa Xaa Xaa Xaa Xaa Lys Arg
1               5                   10                  15

Gly Tyr Phe Leu Phe Arg Pro Arg Asn
            20                  25
```

<210> 5
<211> 25
<212> PRT
<213> Artificial Sequence

<220>
<223> NMU analog

<400> 5

```
Phe Arg Val Asp Glu Glu Phe Gln Ser Pro Phe Ala Ser Gln Lys Arg
1               5                   10                  15

Gly Tyr Phe Leu Phe Arg Pro Arg Asn
            20                  25
```

<210> 6
<211> 25
<212> PRT
<213> Artificial Sequence

<220>
<223> NMU analog

```
<220>
<221>  misc_feature
<222>  (1)..(12)
<223>  Xaa can be any naturally occurring amino acid

<400>  6

Xaa Xaa Xaa Xaa Xaa Xaa Xaa Xaa Xaa Xaa Xaa Xaa Ser Gln Ser Arg
1               5                   10                  15


Gly Tyr Phe Leu Phe Arg Pro Arg Asn
            20                  25


<210>  7
<211>  25
<212>  PRT
<213>  Artificial Sequence

<220>
<223>  NMU analog


<220>
<221>  misc_feature
<222>  (1)..(12)
<223>  Xaa can be any naturally occurring amino acid

<400>  7

Xaa Xaa Xaa Xaa Xaa Xaa Xaa Xaa Xaa Xaa Xaa Xaa Lys Gln Ser Arg
1               5                   10                  15


Gly Tyr Phe Leu Phe Arg Pro Arg Asn
            20                  25


<210>  8
<211>  25
<212>  PRT
<213>  Artificial Sequence

<220>
<223>  NMU analog

<400>  8

Phe Arg Val Asp Glu Glu Phe Gln Ser Pro Phe Ala Lys Gln Ser Arg
1               5                   10                  15


Gly Tyr Phe Leu Phe Arg Pro Arg Asn
            20                  25


<210>  9
<211>  25
<212>  PRT
<213>  Artificial Sequence
```

```
<220>
<223>  NMU analog


<220>
<221>  misc_feature
<222>  (1)..(8)
<223>  Xaa can be any naturally occurring amino acid

<400>  9

Xaa Xaa Xaa Xaa Xaa Xaa Xaa Xaa Ser Pro Phe Ala Ser Gln Ser Arg
1               5                   10                  15


Gly Tyr Phe Leu Phe Arg Pro Arg Asn
            20                  25


<210>  10
<211>  25
<212>  PRT
<213>  Artificial Sequence

<220>
<223>  NMU analog


<220>
<221>  misc_feature
<222>  (1)..(8)
<223>  Xaa can be any naturally occurring amino acid

<400>  10

Xaa Xaa Xaa Xaa Xaa Xaa Xaa Xaa Lys Pro Phe Ala Ser Gln Ser Arg
1               5                   10                  15


Gly Tyr Phe Leu Phe Arg Pro Arg Asn
            20                  25


<210>  11
<211>  25
<212>  PRT
<213>  Artificial Sequence

<220>
<223>  NMU analog

<400>  11

Phe Arg Val Asp Glu Glu Phe Gln Lys Pro Phe Ala Ser Gln Ser Arg
1               5                   10                  15


Gly Tyr Phe Leu Phe Arg Pro Arg Asn
            20                  25


<210>  12
<211>  25
```

```
<212>  PRT
<213>  Artificial Sequence

<220>
<223>  NMU analog


<220>
<221>  misc_feature
<222>  (1)..(5)
<223>  Xaa can be any naturally occurring amino acid

<400>  12

Xaa Xaa Xaa Xaa Xaa Glu Phe Gln Ser Pro Phe Ala Ser Gln Ser Arg
1               5                   10                  15


Gly Tyr Phe Leu Phe Arg Pro Arg Asn
            20                  25


<210>  13
<211>  25
<212>  PRT
<213>  Artificial Sequence

<220>
<223>  NMU analog


<220>
<221>  misc_feature
<222>  (1)..(5)
<223>  Xaa can be any naturally occurring amino acid

<400>  13

Xaa Xaa Xaa Xaa Xaa Lys Phe Gln Ser Pro Phe Ala Ser Gln Ser Arg
1               5                   10                  15


Gly Tyr Phe Leu Phe Arg Pro Arg Asn
            20                  25


<210>  14
<211>  25
<212>  PRT
<213>  Artificial Sequence

<220>
<223>  NMU analog

<400>  14

Phe Arg Val Asp Glu Lys Phe Gln Ser Pro Phe Ala Ser Gln Ser Arg
1               5                   10                  15


Gly Tyr Phe Leu Phe Arg Pro Arg Asn
            20                  25
```

<210> 15
<211> 25
<212> PRT
<213> Artificial Sequence

<220>
<223> NMU analog

<220>
<221> misc_feature
<222> (1)..(3)
<223> Xaa can be any naturally occurring amino acid

<400> 15

```
Xaa Xaa Xaa Asp Glu Glu Phe Gln Ser Pro Phe Ala Ser Gln Ser Arg
1               5                   10                  15

Gly Tyr Phe Leu Phe Arg Pro Arg Asn
            20                  25
```

<210> 16
<211> 25
<212> PRT
<213> Artificial Sequence

<220>
<223> NMU analog

<220>
<221> misc_feature
<222> (1)..(3)
<223> Xaa can be any naturally occurring amino acid

<400> 16

```
Xaa Xaa Xaa Lys Glu Glu Phe Gln Ser Pro Phe Ala Ser Gln Ser Arg
1               5                   10                  15

Gly Tyr Phe Leu Phe Arg Pro Arg Asn
            20                  25
```

<210> 17
<211> 25
<212> PRT
<213> Artificial Sequence

<220>
<223> NMU analog

<400> 17

```
Phe Arg Val Lys Glu Glu Phe Gln Ser Pro Phe Ala Ser Gln Ser Arg
1               5                   10                  15
```

```
Gly Tyr Phe Leu Phe Arg Pro Arg Asn
            20                  25
```